# EUROPEAN PATENT APPLICATION

(11) **EP 2 873 432 A1**
(43) Date of publication of application: **20.05.2015**
(21) Application number: 13192713.9
(22) Date of filing: 13.11.2013
(51) Int. Cl.: A61M 16/04, A61M 39/20, F16L 3/12, A61M 25/02

(54) **Securing assembly of a tracheotomy tube**

(71) Applicant: Vitaltec Corporation, Taichung City (TW)
(72) Inventor: Chiu, Sheng-Yu, Taichung City (TW)
(74) Representative: Pallini Gervasi, Diego

(57) **Abstract**

A securing assembly (1, 1A) of a tracheotomy tube (7) has a cap (10, 10A), a retainer (20, 20A) mounted in the cap (10, 10A), and a connector (30, 30A) attached to the retainer (20, 20). The retainer (20, 20A) has multiple holding portions (22, 22A) mounted around the tracheotomy tube (7). Each holding portion (22, 22A) has a holding part (222, 222A) with a gradually increased thickness. The cap (10, 10A) has multiple pressing protrusions (122, 122A) respectively pressing against the holding parts (222, 222A). When the cap (10, 10A) is turned, the pressing protrusions (122, 122A) force the holding parts (222, 222A) to securely hold the tracheotomy tube (7). The tracheotomy tube (7) would not be released from the securing assembly (1, 1A) easily even though the securing assembly (1, 1A) is inadvertently hit.

## Description

### 1. Field of the Invention

The present invention relates to a securing assembly of a tracheotomy tube, especially to a securing assembly that selectively holds or releases the tracheotomy tube with rotation of a cap.

### 2. Description of the Prior Art(s)

According to medical practice guidelines, when a patient is unable to swallow, to discharge phlegm, or to breathe autonomously, or when respiratory disorders occur, an incision has to be made on an anterior portion of the neck of the patient in order to put a tracheotomy tube into a trachea of the patient through the incision to assist the patient with breathing or to allow the doctor to suck the phlegm out of the trachea of the patient.

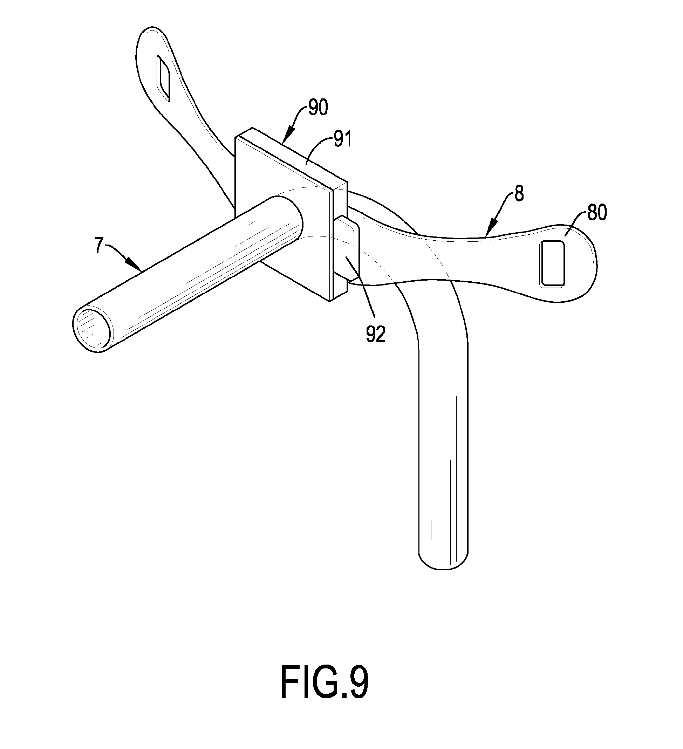

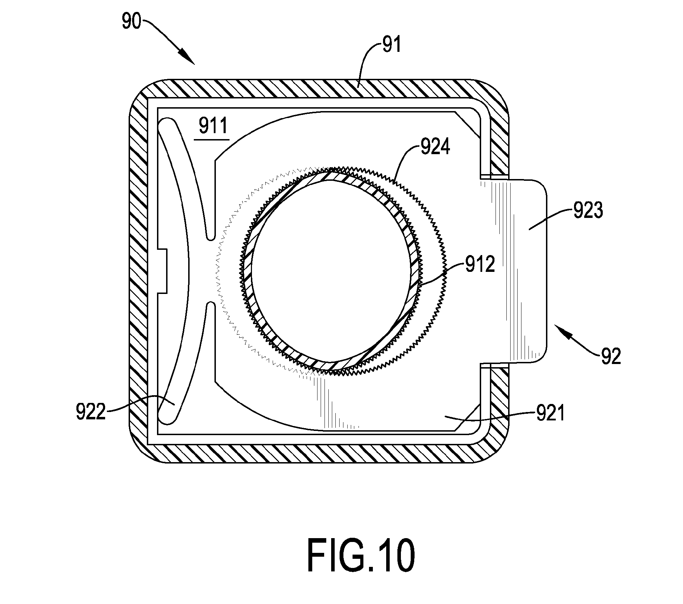

With reference to Figs. 9 and 10, a conventional securing assembly 8 of a tracheotomy tube 7 comprises a clamping portion 90 and two wings 80. The clamping portion 90 has a casing 91 and a retainer 92. The casing 91 is hollow and has a mounting chamber 911, two mounting holes 912, and two hole edges. The mounting chamber 911 is defined in the casing 91. The mounting holes 912 are respectively formed through two opposite walls of the casing 91, align with each other, and communicate with the mounting chamber 911. The hole edges are respectively defined around the mounting holes 912 and are serrate. The retainer 92 is mounted in the mounting chamber 911 of the casing 91 and has a main portion 921, a resilient portion 922 and a pressing portion 923. The main portion 921 is mounted in the mounting chamber 911 of the casing 91 and has a clamping hole 924. The resilient portion 922 and the pressing portion 923 are respectively formed on two opposite sides of the main portion 921. The resilient portion 922 is arced, is disposed in the mounting chamber 911 of the casing 91, and resiliently presses against the casing 91. Thus, the clamping hole 924 misaligns with each of the mounting holes 912 in a normal status. The pressing portion 923 protrudes out of the casing 91. The wings 80 are respectively attached to two opposite sides of the casing 91 of the clamping portion 90 and can be mounted around the neck of the patient, such that the conventional securing assembly 8 of the tracheotomy tube 7 is secured on the patient.

When the pressing portion 923 of the retainer 92 is pressed into the casing 91, the main portion 921 is moved, the resilient portion 922 is compressed, and the clamping hole 924 of the main portion 921 is coaxial with the mounting holes 912 of the casing 91, such that the tracheotomy tube 7 can be mounted through the mounting holes 912 and the clamping hole 924 and inserted into the trachea of the patient. Then, as the pressing portion 923 of the retainer 92 is released, the resilient portion 922 pushes the casing 91 to allow the retainer 92 to move backwardly. Thus, the clamping hole 924 misaligns with each of the mounting holes 912 again, and the tracheotomy tube 7 is clamped in the clamping hole 924 of the retainer 92 and the mounting holes 912 of the casing 91.

However, the tracheotomy tube 7 that is clamped by the conventional securing assembly 8 is released by pressing the retainer 92, and the retainer 92 may be inadvertently hit. Once the tracheotomy tube 7 is released from the clamping portion 90, the tracheotomy tube 7 moves relative to the conventional securing assembly 8 easily, which causes discomfort of the patient.

The main objective of the present invention is to provide a securing assembly of a tracheotomy tube. The securing assembly has a cap, a retainer detachably mounted in the cap, and a connector attached to the retainer. The retainer has an annular seat and multiple holding portions mounted around the tracheotomy tube. Each holding portion has a stationary part and a holding part protruding from the stationary part. The holding part has a proximal side attached to the stationary part and has a holding side opposite to the proximal side. A thickness of the holding part of each holding portion gradually increases from the proximal side to the holding side. The cap has multiple pressing protrusions respectively pressing against the holding parts of the holding portions of the retainer.

When the cap is turned, the pressing protrusions gradually force the holding sides of the holding parts to press against the tracheotomy tube. Thus, the tracheotomy tube is securely held by the holding sides of the holding parts and would not be released from the securing assembly easily even if the securing assembly is inadvertently hit.

### IN THE DRAWINGS:

Fig. 1 is an operational perspective view of a first embodiment of a securing assembly of a tracheotomy tube in accordance with the present invention;
Fig. 2 is an operational exploded perspective view of the securing assembly in Fig. 1;
Fig. 3 is an exploded perspective view of a cap and a rotational retainer of the securing assembly in Fig. 1;
Fig. 4 is an operational enlarged cross-sectional side view of the securing assembly in Fig. 1;
Fig. 5 is an operational and cross-sectional front view of the securing assembly in Fig. 1;
Fig. 6 is another operational and cross-sectional front view of the securing assembly in Fig. 1;
Fig. 7 is an operational exploded perspective view of a second embodiment of a securing assembly of a tracheotomy tube in accordance with the present invention;
Fig. 8 is an operational and cross-sectional front view of the securing assembly in Fig. 7;
Fig. 9 is an operational perspective view of a conventional securing assembly of a tracheotomy tube in accordance with the prior art; and
Fig. 10 is an operational and cross-sectional front view of the conventional securing assembly in Fig. 9.

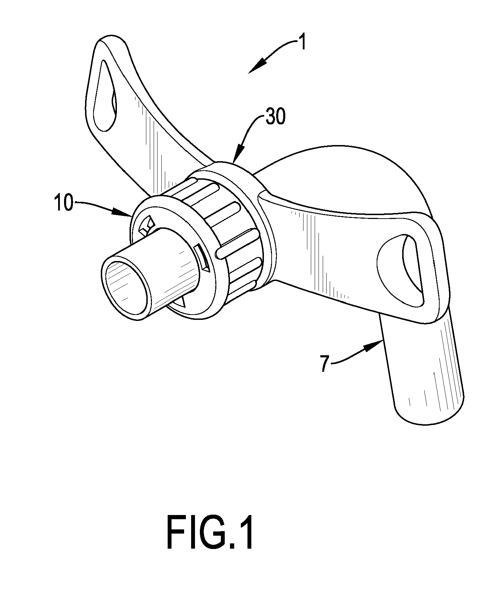

With reference to Figs. 1, 2, and 7, a securing assembly 1, 1A of a tracheotomy tube 7 in accordance with the present invention comprises a cap 10, 10A, a retainer 20, 20A, and a connector 30, 30A.

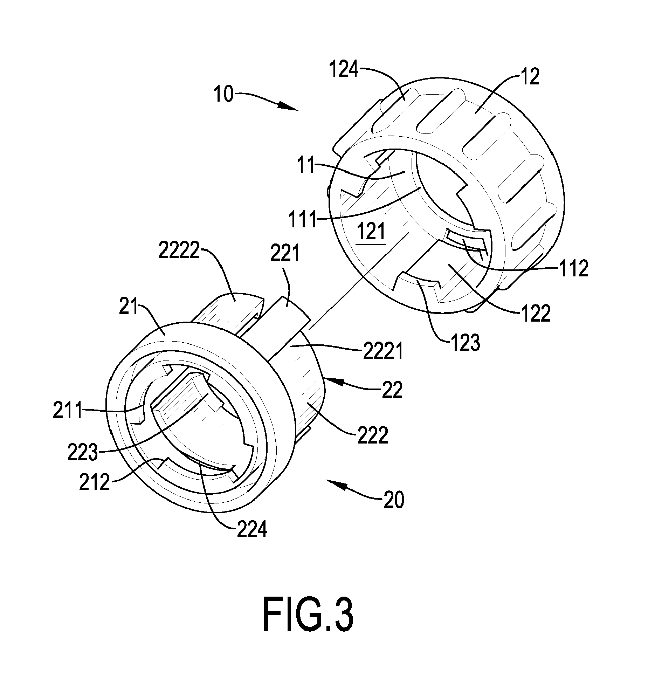

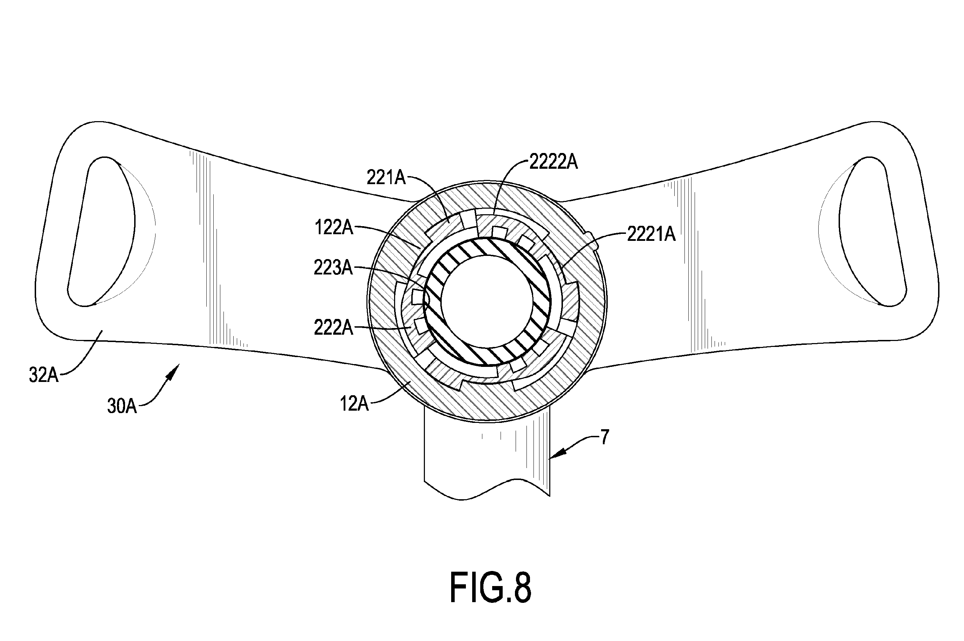

With further reference to Figs. 3 and 8, the cap 10, 10A has an annular sidewall 12, 12A, a front end panel 11, 11A, a mounting chamber 121, 121A, multiple pressing protrusions 122, 122A, multiple engaging protrusions 123, and multiple anti-slipping protrusions 124, 124A.

The annular sidewall 12, 12A of the cap 10, 10A has a front end, a rear end, an outer surface, and an inner surface. The front end panel 11, 11A is formed on the front end of the annular sidewall 12, 12A of the cap 10, 10A and has an insertion hole 111, 111A and multiple through holes 112, 112A. The insertion hole 111, 111A is formed through the front end panel 11, 11A. The through holes 112, 112A are separately formed through the front end panel 11, 11A and are arranged around the insertion hole 111, 111A of the front end panel 11, 11A. The mounting chamber 121, 121A is defined in the cap 10, 10A and is surrounded by the annular sidewall 12, 12A of the cap 10, 10A.

The pressing protrusions 122, 122A are separately formed on the inner surface of the annular sidewall 12, 12A of the cap 10, 10A, are arranged around the mounting chamber 121, 121A, and respectively correspond in position to the through holes 112, 112A of the front end panel 11, 11A. Each pressing protrusion 122, 122A is elongated and extends along an axial direction of the cap 10, 10A. Each pressing protrusion 122, 122A has a front end and a rear end. The front end of the pressing protrusion 122, 122A faces the front end of the annular sidewall 12, 12A of the cap 10, 10A. The rear end of the pressing protrusion 122, 122A faces the rear end of the annular sidewall 12, 12A of the cap 10, 10A. The engaging protrusions 123 are respectively formed on and radially protrude from the pressing protrusions 122, 122A. Each engaging protrusion 123 is disposed adjacent to the rear end of the pressing protrusion 122, 122A. The anti-slipping protrusions 124, 124A are separately formed on the outer surface of the annular sidewall 12, 12A of the cap 10, 10A to allow the cap 10, 10A to be firmly gripped.

The retainer 20, 20A is detachably mounted in the cap 10, 10A and has an annular seat 21, 21A and multiple holding portions 22, 22A.

The annular seat 21, 21A is circular and ring-shaped and has a rear end surface, a front end surface, a mounting hole 211, and multiple positioning recesses 212. The mounting hole 211 of the annular seat 21, 21A is formed through the annular seat 21, 21A. The positioning recesses 212 are separately formed in the rear end surface of the annular seat 21, 21A and are arranged around the mounting hole 211 of the annular seat 21, 21A.

The holding portions 22, 22A are formed on the front end surface of the annular seat 21, 21A, are arranged around the mounting hole 211 of the annular seat 21, 21A, and are mounted into the mounting chamber 121, 121A of the cap 10, 10A from the rear end of the annular sidewall 12, 12A of the cap 10, 10A. Each of the holding portions 22, 22A has a stationary part 221, 221A and a holding part 222, 222A. The stationary part 221, 221A is elongated, axially protrudes from the front end surface of the annular seat 21, 21A, and has an elongated side perpendicular to the front end surface of the annular seat 21, 21A. The holding part 222, 222A is arced, is formed on and protrudes form the elongated side of the stationary part 221, 221A, and is disposed apart from the annular seat 21, 21A to form a gap 224, 224A defined between the holding part 222, 222A and the annular seat 21, 21A. Thus, the holding part 222, 222A can selectively move relative to the annular seat 21, 21A. The holding part 222, 222A has an inner surface, an outer surface, a proximal side 2221, 2221A and a holding side 2222, 2222A. The outer surface of the holding part 222, 222A faces the annular sidewall 12, 12A of the cap 10, 10A. The proximal side 2221, 2221A of the holding part 222, 222A is attached to the elongated side of the stationary part 221, 221A. The holding side 2222, 2222A of the holding part 222, 222A is opposite to the proximal side 2221, 2221A of the holding part 222, 222A. A thickness of the holding part 222, 222A gradually increases from the proximal side 2221, 2221A to the holding side 2222, 2222A.

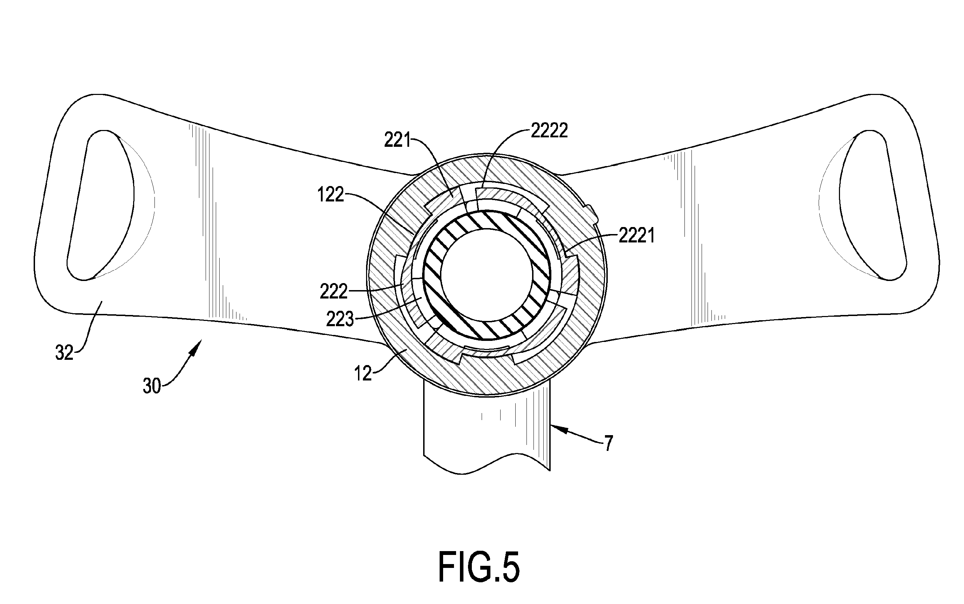

With further reference to Fig. 5, when mounting the retainer 20, 20A into the cap 10, 10A, the pressing protrusions 122, 122A of the cap 10, 10A respectively press against the outer surfaces of the holding parts 222, 222A of the holding portions 22, 22A of the retainer 20, 20A. Moreover, the engaging protrusions 123 of the cap 10, 10A respectively engage in the gaps 224, 224A defined between the holding parts 222, 222A and the annular seat 21, 21A, such that the cap 10, 10A is securely mounted on the retainer 20, 20A.

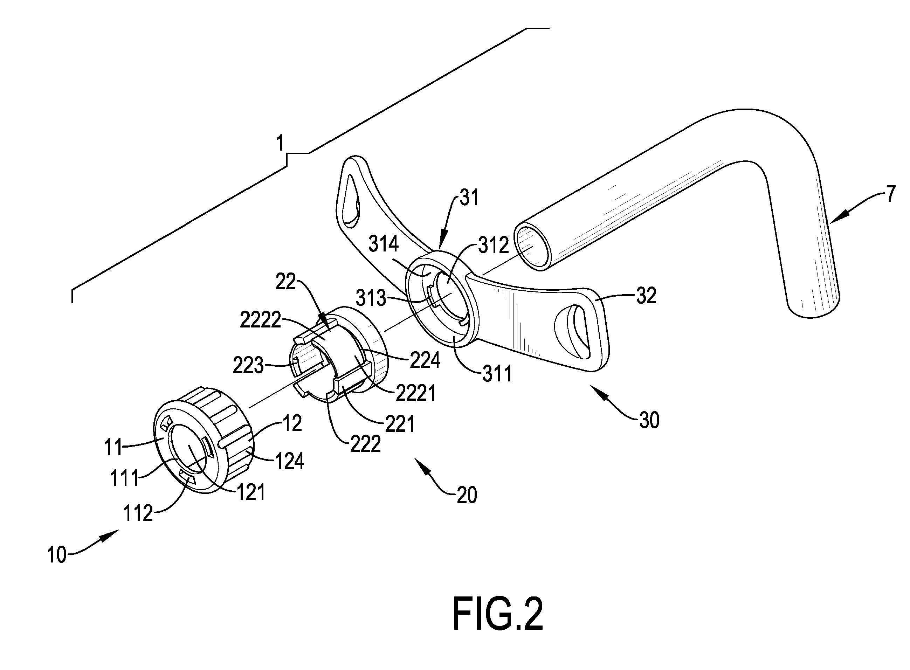

As shown in Fig. 2, in a first embodiment of the present invention, the holding part 222 of each holding portion 22 further has at least one holding protrusion 223 formed on and radially protruding from the inner surface of the holding part 222 and disposed adjacent to the holding side 2222 of the holding part 222.

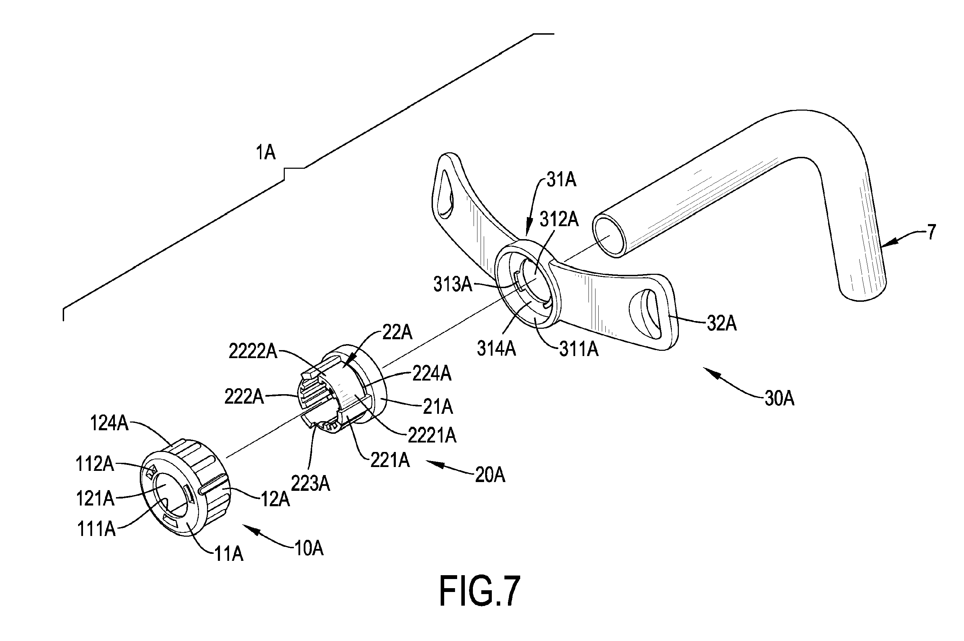

As shown in Fig. 7, in a second embodiment of the present invention, the inner surface of the holding part 222A of each holding portion 22A is bumpy.

The connector 30, 30A is attached to the annular seat 21, 21A of the retainer 20, 20A and has a positioning seat 31, 31A and two wings 32, 32A.

The positioning seat 31, 31A is attached to the annular seat 21, 21A of the retainer 20, 20A and has an annular sidewall 311, 311A, a rear end panel 314, 314A, and multiple positioning protrusions 313, 313A. The annular sidewall 311, 311A of the positioning seat 31, 31A is mounted around the annular seat 21, 21A of the retainer 20, 20A and has a front end and a rear end. The rear end panel 314, 314A is formed on the rear end of the annular sidewall 311, 311A of the positioning seat 31, 31A and has a mounting hole 312, 312A and a hole edge. The mounting hole 312, 312A of the rear end panel 314, 314A is formed through the rear end panel 314, 314A. The hole edge is defined around the mounting hole 312, 312A of the rear end panel 314, 314A. The positioning protrusions 313, 313A are separately formed on the rear end panel 314, 314A, are arranged around the mounting hole 312, 312A of the rear end panel 314, 314A, protrude toward the front end of the annular sidewall 311, 311A of the positioning seat 31, 31A, and respectively engage in the positioning recesses 212 of the annular seat 21, 21A.

The wings 32, 32A are oppositely disposed. Specifically, the wings 32, 32A are attached to and protrude from the annular sidewall 311, 311A of the positioning seat 31, 31A and are disposed opposite to each other.

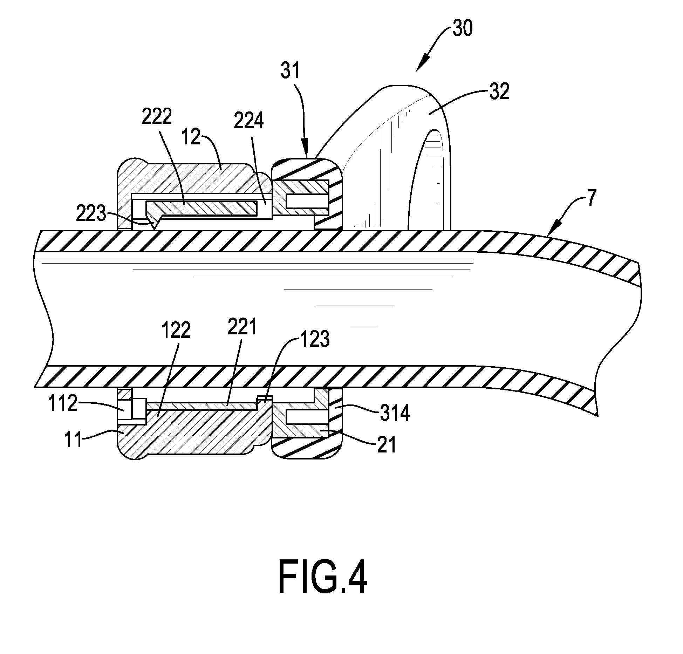

With reference to Figs. 4, 5, and 8, the tracheotomy tube 7 is mounted through the insertion hole 111, 111A of the cap 10, 10A, the mounting hole 211 of the annular seat 21, 21A, and the mounting hole 312 of the connector 30, 30A. As shown in Figs. 5 and 8, as the pressing protrusions 122, 122A of the cap 10, 10A respectively correspond in position to the proximal sides 2221, 2221A of the holding parts 222, 222A, the tracheotomy tube 7 is released from the holding parts 222, 222A. Thus, relative positions of the tracheotomy tube 7 and the securing assembly 1, 1A can be adjusted.

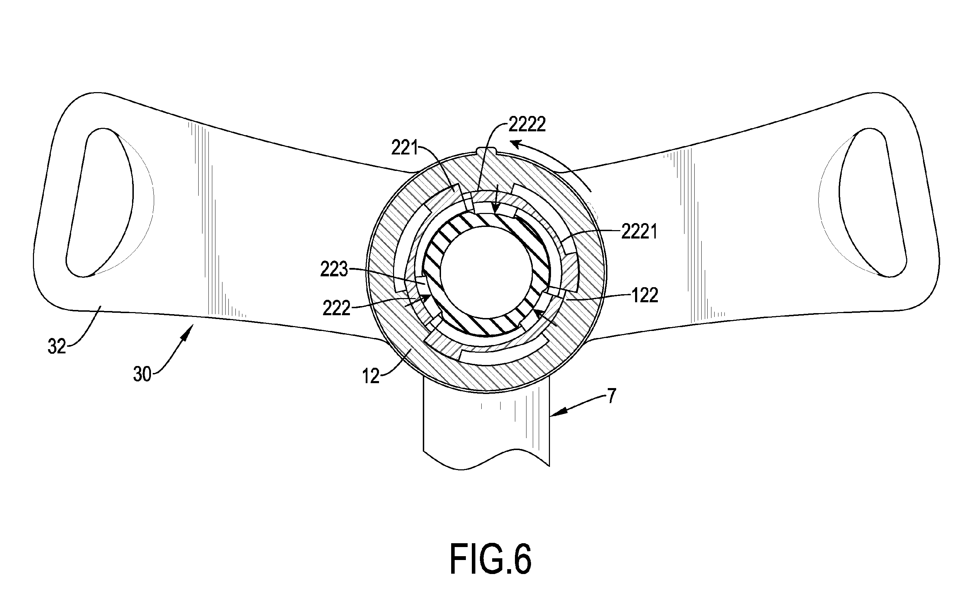

With further reference to Fig. 6, since the thickness of each holding part 222, 222A increases gradually from the proximal side 2221, 2221A to the holding side 2222, 2222A, as the cap 10, 10A is turned to allow the pressing protrusions 122, 122A of the cap 10, 10A to move toward the holding sides 2222, 2222A of the holding parts 222, 222A, the pressing protrusions 122, 122A gradually force the holding sides 2222, 2222A of the holding parts 222, 222A to press against the tracheotomy tube 7. Thus, the tracheotomy tube 7 is securely held by the holding sides 2222, 2222A of the holding parts 222, 222A. Since the tracheotomy tube 7 is selectively held or released with rotation of the cap 10, 10A, the tracheotomy tube 7 would not be released from the securing assembly (1, 1A) easily even if the securing assembly (1, 1A) is inadvertently hit.

## Claims

1. A securing assembly (1, 1A) of a tracheotomy tube (7) comprising a connector (30, 30A) having two wings (32) oppositely disposed, and the securing assembly (1, 1A) **characterized in that**:
the securing assembly (1, 1A) further comprises
a cap (10, 10A) having
an annular sidewall (12, 12A);
a mounting chamber (121, 121A) surrounded by the annular sidewall (12, 12A) of the cap (10, 10A); and
multiple pressing protrusions (122, 122A) separately formed on an inner surface of the annular sidewall (12, 12A) of the cap (10, 10A) and arranged around the mounting chamber (121, 121A); and
a retainer (20, 20A) detachably mounted in the cap (10, 10A) and having
an annular seat (21, 21A) having a front end surface and a mounting hole (211); and
multiple holding portions (22, 22A) formed on the front end surface of the annular seat (21, 21A), arranged around the mounting hole (211) of the annular seat (21, 21A), and mounted in the mounting chamber (121, 121A) of the cap (10, 10A), and each of the holding portions (22, 22A) having
a stationary part (221, 221A) axially protruding from the front end surface of the annular seat (21, 21A); and
a holding part (222, 222A) formed on and protruding form the stationary part (221, 221A) and having
an outer surface facing the annular sidewall (12, 12A) of the cap (10, 10A);
a proximal side (2221, 2221A) attached to the stationary part (221, 22 1 A); and
a holding side (2222, 2222A) opposite to the proximal side (2221, 2221A) of the holding part (222, 222A);
the connector (30, 30A) is attached to the annular seat (21, 21A) of the retainer (20, 20A);
wherein a thickness of the holding part (222, 222A) of each holding portion (22, 22A) gradually increases from the proximal side (2221, 2221A) to the holding side (2222, 2222A); and
the pressing protrusions (122, 122A) of the cap (10, 10A) respectively press against the outer surfaces of the holding parts (222, 222A) of the holding portions (22, 22A) of the retainer (20, 20A).

2. The securing assembly (1, 1A) as claimed in claim 1, wherein
the cap (10, 10A) further has a front end panel (11, 11A) formed on a front end of the annular sidewall (12, 12A) of the cap (10, 10A) and having an insertion hole (111, 111A); and
the stationary part (221, 221A) of each holding portion (22, 22A) of the retainer (20, 20A) has an elongated side perpendicular to the front end surface of the annular seat (21, 21A);
the holding part (222, 222A) of each holding portion (22, 22A) of the retainer (20, 20A) is formed on and protrudes form the elongated side of the stationary part (221, 221A), and is disposed apart from the annular seat (21, 21A) to form a gap (224, 224A) defined between the holding part (222, 222A) and the annular seat (21, 21A), such that the holding part (222, 222A) can selectively move relative to the annular seat (21, 21A);
the connector (30, 30A) further has a positioning seat (31, 31A) attached to the annular seat (21, 21A) of the retainer (20, 20A) and having
an annular sidewall (311, 311A) mounted around the annular seat (21, 21A) of the retainer (20, 20A); and
a rear end panel (314, 314A) formed on a rear end of the annular sidewall (311, 311A) of the positioning seat (31, 31A) and having a mounting hole (312, 312A); and
the wings (32, 32A) of the connector (30, 30A) are attached to and protrude from the annular sidewall (311, 311A) of the positioning seat (31, 31A).

3. The securing assembly (1) as claimed in claim 2, wherein the holding part (222) of each holding portion (22) of the retainer (20) further has at least one holding protrusion (223) formed on and radially protruding from an inner surface of the holding part (222) and disposed adjacent to the holding side (2222) of the holding part (222).

4. The securing assembly (1A) as claimed in claim 2, wherein an inner surface of the holding part (222A) of each holding portion (22A) of the retainer (20A) is bumpy.

5. The securing assembly (1, 1A) as claimed in claim 2, 3, or 4, wherein
each pressing protrusion (122, 122A) of the cap (10, 10A) has a rear end and a front end facing the front end panel (11, 11A) of the cap (10, 10A); and
the cap (10, 10A) further has multiple engaging protrusions (123) respectively formed on and radially protruding from the pressing protrusions (122, 122A) of the cap (10, 10A), and each engaging protrusion (123) is disposed adjacent to the rear end of the pressing protrusion (122, 122A) and respectively engages in the gaps (24, 24A) defined between the holding parts (222, 222A) and the annular seat (21, 21A).

6. The securing assembly (1, 1A) as claimed in claim 5, wherein
the annular seat (21, 21A) of the retainer (20, 20A) further has multiple positioning recesses (212) separately formed in a rear end surface of the annular seat (21, 21A) and arranged around the mounting hole (211) of the annular seat (21, 21A); and
the positioning seat (31, 31A) of the connector (30, 30A) further has multiple positioning protrusions (313, 313A) separately formed on the rear end panel (314, 314A) of the positioning seat (31, 31A), arranged around the mounting hole (312, 312A) of the rear end panel (314, 314A), protruding toward a front end of the annular sidewall (311, 311A) of the positioning seat (31, 31A), and respectively engaging in the positioning recesses (212) of the annular seat (21, 21A).

7. The securing assembly (1, 1A) as claimed in claim 6, wherein the front end panel (11, 11A) of the cap (10, 10A) further has multiple through holes (112, 112A) separately formed through the front end panel (11, 11A), arranged around the insertion hole (111, 111A) of the front end panel (11, 11A), and respectively corresponding in position to the pressing protrusions (122, 122A) of the cap (10, 10A).

8. The securing assembly (1, 1A) as claimed in claim 7, wherein the cap (10, 10A) further has multiple anti-slipping protrusions (124, 124A) separately formed on an outer surface of the annular sidewall (12, 12A) of the cap (10, 10A).
